(19)

Europäisches
Patentamt
European
Patent Office
Office européen
des brevets

(11) **EP 4 606 316 A1**

(12) **EUROPEAN PATENT APPLICATION**
published in accordance with Art. 153(4) EPC

(43) Date of publication:
**27.08.2025 Bulletin 2025/35**

(21) Application number: **23893699.1**

(22) Date of filing: **15.11.2023**

(51) International Patent Classification (IPC):
***A61B 8/00*** (2006.01)

(52) Cooperative Patent Classification (CPC):
**A61B 8/00**

(86) International application number:
**PCT/CN2023/131868**

(87) International publication number:
**WO 2024/109609 (30.05.2024 Gazette 2024/22)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **25.11.2022 CN 202211487363**

(71) Applicant: **Wuhan United Imaging Healthcare Co.,
Ltd.
WuHan, Hubei 430206 (CN)**

(72) Inventors:
• **SHI, Aiwei
Wuhan, Hubei 430206 (CN)**
• **ZHANG, Jianmin
Wuhan, Hubei 430206 (CN)**

(74) Representative: **Wang, Bo
Panovision IP
Ebersberger Straße 3
85570 Markt Schwaben (DE)**

(54) **ULTRASONIC COMPOSITE IMAGING METHOD, SYSTEM AND APPARATUS, AND STORAGE
MEDIUM**

(57)     Provided are a method, system, and device for ultrasonic composite imaging, and a storage medium thereof. The method includes obtaining a beamforming result sequence (310) of at least one pixel point in an imaging region, the beamforming result sequence including beamforming results that are arranged according to their respective emission orders; determining a composite weight sequence (320) based on a tissue movement speed of the at least one pixel point; and determining, based on the beamforming result sequence and the composite weight sequence, a composite result to generate an ultrasound image (330) of the imaging region.

**300**

Obtaining a beamforming result sequence of at least one pixel point in an imaging region — 310

↓

Determining a composite weight sequence based on a tissue movement speed of the at least one pixel point — 320

↓

Determining, based on the beamforming result sequence and the composite weight sequence, a composite result to generate an ultrasound image of the imaging region — 330

**FIG. 3**

**EP 4 606 316 A1**

**Description**

**CROSS-REFERENCE TO RELATED APPLICATIONS**

This application claims priority of Chinese Patent Application No. 202211487363.3, filed on November 25, 2022, the entire contents of each of which are hereby incorporated by reference.

**TECHNICAL FIELD**

The present disclosure relates to the field of medical imaging, and in particular, to methods, systems, devices, and storage media for ultrasonic composite imaging.

**BACKGROUND**

Ultrasound imaging is one of the most extensively utilized medical imaging technologies. This technology involves the emission of ultrasound waves from a probe into a target object, followed by the generation of an image based on echo signals of the ultrasound waves. Compared with other imaging technologies, ultrasound imaging has a lower signal-to-noise ratio. Composite imaging technology, such as coherent composite and incoherent composite, has been widely adopted to enhance the signal-to-noise ratio of ultrasound images. However, the composite imaging technology is significantly compromised by tissue movement relative to the transducer, leading to a substantial degradation in image resolution and affecting the imaging quality.

Therefore, it is desirable to provide a method, a system, a device, and a storage medium for ultrasonic composite imaging, which may utilize composite imaging technology to improve image resolution while ensuring the accuracy of imaging in moving tissue regions.

**SUMMARY**

One of the embodiments of the present disclosure provides a method for ultrasonic composite imaging. The method for ultrasonic composite imaging may include obtaining a beamforming result sequence of at least one pixel point in an imaging region, the beamforming result sequence including beamforming results that are arranged according to their respective emission orders; determining a composite weight sequence based on a tissue movement speed of the at least one pixel point; and determining, based on the beamforming result sequence and the composite weight sequence, a composite result to generate an ultrasound image of the imaging region.

In some embodiments, a processing device may determine a speed level of the tissue movement speed of the at least one pixel point and determine, based on the speed level, the composite weight sequence.

In some embodiments, the processing device may divide, based on plane wave data in the imaging region, the imaging region into at least one sub-region and determine a speed level of the at least one sub-region; and determine the speed level of the sub-region where the at least one pixel point is located as the speed level of the tissue movement speed of the at least one pixel point.

In some embodiments, the processing device may determine a frequency shift of each of the at least one pixel point in the imaging region based on the plane wave data in the imaging region; preset at least one frequency shift threshold; divide, based on the at least one frequency shift threshold and a proportional relationship between the frequency shift and the tissue movement speed, the imaging region into the at least one sub-region.

In some embodiments, for each pixel point, the processing device may obtain a plurality of pieces of plane wave data via a plurality of emissions, determine a plurality of initial frequency shifts based on the Doppler theorem, and determine an average value of the plurality of initial frequency shifts as the frequency shift of the pixel point.

In some embodiments, the composite weight sequence may be determined based on Gaussian distributions. The processing device may determine a variance of at least one of the Gaussian distributions based on the speed level of the at least one sub-region; generate the composite weight sequence of the at least one sub-region based on the variance of the at least one Gaussian distribution; and determine the composite weight sequence of the at least one pixel point according to the sub-region where the at least one pixel point is located.

In some embodiments, the variance of the at least one Gaussian distribution may be positively correlated with the speed level.

In some embodiments, the processing device may obtain a plurality of reference ultrasound images by emitting a focused wave targeted at the imaging region; determine tissue movement information between adjacent reference ultrasound images based on a correspondence between the adjacent reference ultrasound images; divide the imaging region into at least one sub-region based on the tissue movement information and determine the speed level of the tissue movement speed of the at least one pixel point based on a sub-region, among the at least one sub-region, where the at least one pixel point is located.

In some embodiments, the processing device may determine a target beamforming result based on the beamforming result sequence, the target beamforming result being a beamforming result in the beamforming result sequence that satisfies a first preset condition; determine a target weight in the composite weight sequence based on a Gaussian distribution, the target weight being a weight that satisfies a second preset condition; determine a weight allocation result by allocating the target weight and weights on two sides of the target weight to the target beamforming result and beamforming results on two sides of the target beamforming result, respectively; determine a composite result by compositing the beamforming result sequence based on the weight allocation result, and generate the ultrasound image based on the composite result.

In some embodiments, the beamforming result in the beamforming result sequence that satisfies a first preset condition may include a beamforming result in the beamforming result sequence that corresponds to an emission perpendicular to the at least one pixel point.

In some embodiments, the processing device may emit a focused wave targeted at the imaging region for multiple times; for each pixel point in the at least one pixel point, determine at least one beamforming result based on at least one emission of the focused wave and generating the beamforming result sequence of the pixel point based on the at least one beamforming result.

One of the embodiments of the present disclosure provides a system for ultrasonic composite imaging. The system for ultrasonic composite imaging may include an acquisition module, a determination module, and a generation module. The acquisition module may be configured to obtain a beamforming result sequence of at least one pixel point in an imaging region. The beamforming result sequence includes beamforming results that are arranged according to their respective emission orders. The determination module may be configured to determine a composite weight sequence based on a tissue movement speed of the at least one pixel point. The generation module may be configured to determine, based on the beamforming result sequence and the composite weight sequence, a composite result to generate an ultrasound image of the imaging region.

In some embodiments, the determination module may be further configured to determine a speed level of the tissue movement speed of the at least one pixel point and determine, based on the speed level, the composite weight sequence.

In some embodiments, the determination module may be further configured to divide, based on plane wave data in the imaging region, the imaging region into at least one sub-region and determine a speed level of the at least one sub-region and determine the speed level of the sub-region where the at least one pixel point is located as the speed level of the tissue movement speed of the at least one pixel point.

In some embodiments, the determination module may be further configured to determine a frequency shift of each of the at least one pixel point in the imaging region based on the plane wave data in the imaging region; preset at least one frequency shift threshold; and divide, based on the at least one frequency shift threshold and a proportional relationship between the frequency shift and the tissue movement speed, the imaging region into the at least one sub-region.

In some embodiments, the determination module may be further configured to for each pixel point, obtain a plurality of pieces of plane wave data via a plurality of emissions and determine a plurality of initial frequency shifts based on the Doppler theorem; determine an average value of the plurality of initial frequency shifts as the frequency shift of the pixel point.

In some embodiments, the composite weight sequence may be determined based on Gaussian distributions. The determination module may be further configured to determine a variance of at least one of the Gaussian distributions based on the speed level of the at least one sub-region; generate the composite weight sequence of the at least one sub-region based on the variance of the at least one Gaussian distribution.

In some embodiments, the variance of the at least one Gaussian distribution may be positively correlated with the speed level.

In some embodiments, the determination module may be further configured to obtain a plurality of frames of reference ultrasound images by emitting a focused wave targeted at the imaging region; determine tissue movement information between adjacent frames of reference ultrasound images based on a correspondence between the adjacent frames of reference ultrasound images; divide the imaging region into at least one sub-region based on the tissue movement information and determine a speed level of the at least one sub-region; determine the speed level of the tissue movement speed of the at least one pixel point based on a sub-region, among the at least one sub-region, where the at least one pixel point is located.

In some embodiments, the generation module may be further configured to determine a target beamforming result based on the beamforming result sequence, the target beamforming result being a beamforming result in the beamforming result sequence that satisfies a first preset condition; determine a target weight in the composite weight sequence based on a Gaussian distribution, wherein the target weight is a weight that satisfies a second preset condition; determine a weight allocation result by allocating the target weight and weights on two sides of the target weight to the target beamforming result and beamforming results on two sides of the target beamforming result, respectively; determine the composite result by compositing the beamforming result sequence based on the weight allocation result, and generate the ultrasound image based on the composite result.

In some embodiments, the acquisition module may be further configured to emit a focused wave targeted at the imaging region for multiple times; for each pixel point in the at least one pixel point, determine at least one beamforming result based on at least one emission of the focused wave, and generate the beamforming result sequence of the pixel point based on the at least one beamforming result.

One of the embodiments of the present disclosure provides a device for ultrasonic composite imaging, including at least one processor. The at least one processor may be used to execute the method for ultrasonic composite imaging.

One of the embodiments of the present disclosure provides a non-transitory computer-readable storage medium storing computer instructions that, when read by a computer, the computer to perform the method for ultrasonic composite imaging.

**BRIEF DESCRIPTION OF THE DRAWINGS**

The present disclosure is further illustrated in terms of exemplary embodiments. These exemplary embodiments are described in detail with reference to the drawings. These embodiments are non-limiting exemplary embodiments, in which like reference numerals represent similar structures, and wherein:

FIG. 1 is a schematic diagram illustrating a system for ultrasonic composite imaging according to some embodiments of the present disclosure;
FIG. 2 is a block diagram illustrating an exemplary system for ultrasonic composite imaging according to some embodiments of the present disclosure;
FIG. 3 is a flowchart illustrating an exemplary process for ultrasonic composite imaging according to some embodiments of the present disclosure;
FIG. 4 is a schematic diagram illustrating an exemplary focused wave according to some embodiments of the present disclosure;
FIG. 5 is a schematic diagram illustrating an exemplary emission sequence according to some embodiments of the present disclosure;
FIG. 6 is a schematic diagram illustrating an exemplary process for obtaining a beamforming result corresponding to a single emission of a pixel according to some embodiments of the present disclosure;
FIG. 7 is a schematic diagram illustrating an exemplary process for ultrasonic composite imaging according to some embodiments of the present disclosure;
FIG. 8 is a flowchart illustrating an exemplary process for determining a composite weight sequence according to some embodiments of the present disclosure;
FIG. 9 is a schematic diagram illustrating an exemplary plane wave according to some embodiments of the present disclosure;
FIG. 10 is a flowchart illustrating an exemplary process for generating an ultrasound image of an imaging region according to some embodiments of the present disclosure;
FIG. 11 is a flowchart illustrating an exemplary process for obtaining an ultrasound image based on an emission sequence according to some embodiments of the present disclosure; and
FIG. 12 is a schematic diagram illustrating an exemplary computer device according to some embodiments of the present disclosure.

**DETAILED DESCRIPTION**

To more clearly illustrate the technical solutions related to the embodiments of the present disclosure, a brief introduction of the drawings referred to the description of the embodiments is provided below. Obviously, the drawings described below are only some examples or embodiments of the present disclosure. Those having ordinary skills in the art, without further creative efforts, may apply the present disclosure to other similar scenarios according to these drawings. Unless obviously obtained from the context or the context illustrates otherwise, the same numeral in the drawings refers to the same structure or operation.

It should be understood that "system," "device," "unit," and/or "module" as used herein is a manner used to distinguish different components, elements, parts, sections, or assemblies at different levels. However, if other words serve the same purpose, the words may be replaced by other expressions.

As shown in the present disclosure and claims, the words "one," "a," "a kind," and/or "the" are not especially singular but may include the plural unless the context expressly suggests otherwise. In general, the terms "comprise," "comprises," "comprising," "include," "includes," and/or "including," merely prompt to include operations and elements that have been clearly identified, and these operations and elements do not constitute an exclusive listing. The methods or devices may also include other operations or elements.

The flowcharts used in the present disclosure illustrate operations that systems implement according to some embodiments of the present disclosure. It should be understood that the previous or subsequent operations may not be accurately implemented in order. Instead, each step may be processed in reverse order or simultaneously. Meanwhile, other operations may also be added to these processes, or a certain step or several steps may be removed from these processes.

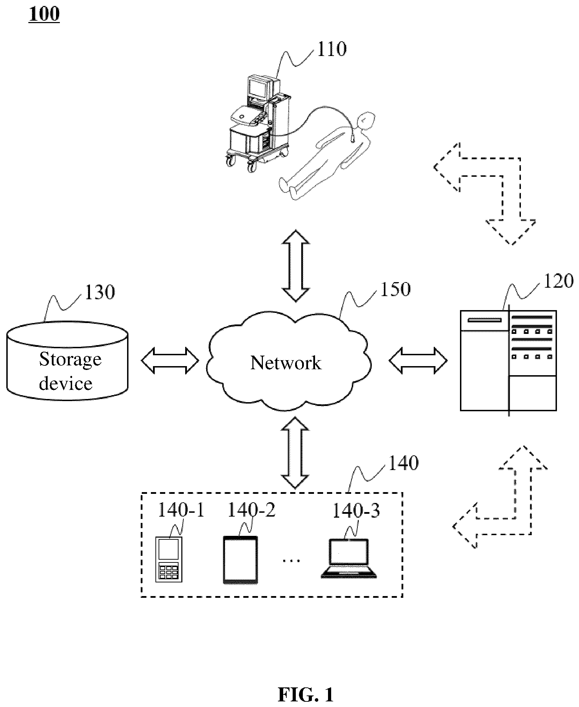

FIG. 1 is a schematic diagram illustrating a system for ultrasonic composite imaging according to some embodiments of the present disclosure.

As shown in FIG. 1, an application scenario 100 of the system for ultrasonic composite imaging may include an ultrasound device 110, a processing device 120, a storage device 130, a terminal 140, and a network 150.

The ultrasound device 110 may be configured to perform a scan on an imaging region to obtain scanning data of the imaging region. The ultrasound device 110 may be configured to view image information of the internal tissue of the imaging region to assist doctors in diagnosing diseases. The ultrasound device 110 may send high frequency sound waves (such as ultrasound waves) to the imaging region through a probe to generate an ultrasound image. In some embodiments, the imaging region may include an imaging region of a biological object and/or an imaging region of a non-biological object. For example, the imaging region may include a specific part of the human body, such as a combination of one or more of the neck, chest, abdomen, etc. In some embodiments, the scanning data related to the imaging region may include plane wave data, focused wave data, etc.

In some embodiments, the ultrasound device 110 may include an ultrasound pulse echo imaging device, an ultrasound echo Doppler imaging device, an ultrasound electronic endoscope, an ultrasound Doppler blood flow analysis device, an ultrasound human tissue measurement device, etc. In some embodiments, the ultrasound image may include at least one of a brightness mode (B mode) image, a color mode (C mode) image, a movement mode (M mode) image, a Doppler mode (D mode) image, and an elastic imaging mode (E mode) image. In some embodiments, the ultrasound image may include a two-dimensional (2D) image or a three-dimensional (3D) image.

In some embodiments, the ultrasound device 110 may transmit a plane wave and a focused wave. The plane wave is used to divide the imaging region into at least one sub-region and determine a speed level of at least one sub-region, and/or determine a composite result. The focused wave is used to determine the composite result to generate the ultrasound image of the imaging region.

In some embodiments, the ultrasound device 110 may also receive imaging region information and/or imaging operation instruction information sent from the terminal 140 or the processing device 120 through the network 150 and send intermediate imaging result data or imaging images to the processing device 120, the storage device 130, or the terminal 140.

The processing device 120 may process data and/or information obtained and/or extracted from the ultrasound device 110, the storage device 130, the terminal 140 and/or other storage devices. In some embodiments, the processing device 120 may obtain a beamforming result sequence of at least one pixel point in the imaging region, and the beamforming result sequence includes beamforming results that are arranged according to their respective emission orders. Further, the processing device 120 may determine a composite weight sequence based on a tissue movement speed of the at least one pixel point, and determine, based on the beamforming result sequence and the composite weight sequence, a composite result, to generate the ultrasound image of the imaging region.

In some embodiments, the processing device 120 may be a single server or a server group. The server group may be centralized or distributed. In some embodiments, the processing device 120 may be local or remote. For example, the processing device 120 may access the information and/or data stored in the ultrasound device 110, the storage device 130, and/or the terminal 140 through the network 150. As another example, the processing device 120 may be directly connected to the ultrasound device 110, the storage device 130, and/or the terminal 140 to access the information and/or data stored therein. In some embodiments, the processing device 120 may be implemented on a cloud platform. Merely by way of example, the cloud platform may include a private cloud, a public cloud, a hybrid cloud, a community cloud, a distributed cloud, an internal cloud, a multi-layer cloud, etc., or any combination thereof.

In some embodiments, the processing device 120 may be implemented on a computing device. The computing device may be a computer connected to the ultrasound device 110, for example, a laptop or desktop computer placed in a scanning room or an operating room. In some embodiments, the ultrasound device 110, the terminal 140, and/or other system components may include the processing device 120. For example, the processing device 120 or a module that can implement the functions of the processing device 120 may be integrated into the ultrasound device 110, the terminal 140, and/or other system components.

The storage device 130 may be configured to store data and/or instructions. In some embodiments, the storage device 130 may store data obtained from the processing device 120 and/or the terminal 140. For example, the storage device 130 may store the plane wave data, the focused wave data, etc. In some embodiments, the storage device 130 may store data and/or instructions, and the processing device 120 may execute or use the data and/or instructions to perform the exemplary methods described in the present disclosure.

In some embodiments, the storage device 130 may include one or more storage assemblies, each of which may be an independent device or part of other devices. In some embodiments, the storage device 130 may include a random-access memory (RAM), a read-only memory (ROM), a mass storage, a removable memory, a volatile read-write memory, or the like, or any combination thereof. For example, the mass storage may include a magnetic disk, an optical disk, a solid-state disk, or the like. In some embodiments, the storage device 130 may be implemented on a cloud platform. In some embodiments, the storage device 130 may be part of the processing device 120.

The terminal 140 may be connected to the ultrasound device 110 and/or the processing device 120 for inputting or outputting information and/or data. For example, the user may issue an operation instruction to the ultrasound device 110 through the terminal 140 so that the ultrasound device 110 performs a specified operation, for example, imaging a specified body part of a patient. In some embodiments, the terminal 140 may instruct the processing device 120 to perform a method for ultrasonic composite imaging as illustrated in some embodiments of the present disclosure. In some embodiments, the composite ultrasound image may be presented to the user through the terminal 140. In some embodiments, the terminal 140 may be one of a mobile device 140-1, a tablet computer 140-2, a laptop computer 140-3, and other devices with input and/or output functions, or any combination thereof. In some embodiments, the terminal 140 may be a part of the processing device 120. In some embodiments, the terminal 140 may be a part of the ultrasound device 110.

The network 150 may connect the components of the system and/or connect the system with external resources. The network 150 enables communication between the components and other components outside the system to facilitate the exchange of data and/or information. In some embodiments, the network 150 may be any one or more of a wired network or a wireless network. For example, the network 150 may include a public network (e.g., the Internet), a private network (e.g., a local area network (LAN), a wide area network (WAN), etc.), a wired network (e.g., Ethernet), a wireless network (e.g., a Wi-Fi network, etc.), a cellular network, a frame relay network, a virtual private network, a satellite network, a telephone network, a router, a hub, a switch, a server computer, and/or any combination thereof. The network connection between the components may be in one of the above-mentioned ways or in a plurality of ways. In some embodiments, the network 150 may include one or more network access points. For example, the network 150 may include a wired or wireless network access point, through which one or more components in the application scenario 100 of the system for ultrasonic composite imaging may be connected to the network 150 to exchange data and/or information.

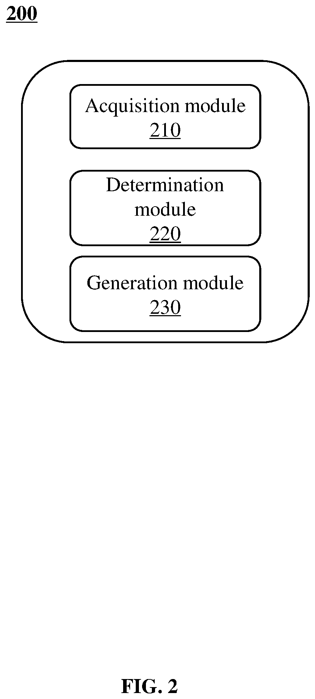

FIG. 2 is a block diagram illustrating an exemplary system for ultrasonic composite imaging according to some embodiments of the present disclosure. As shown in FIG. 2, a system for ultrasonic composite imaging 200 may include an acquisition module 210, a determination module 220, and a generation module 230. In some embodiments, the system for ultrasonic composite imaging 200 may be configured on a processing device (e.g., the processing device 120) to perform the functions of each module of the system for ultrasonic composite imaging 200.

In some embodiments, the acquisition module 210 may be configured to obtain a beamforming result sequence of at least one pixel point in an imaging region.

In some embodiments, the acquisition module 210 may be further configured to emit a focused wave targeted at the imaging region for multiple times, for each pixel point in the at least one pixel point, determine at least one beamforming result based on at least one emission of the focused wave, and generate a beamforming result sequence of the pixel point based on the at least one beamforming result.

In some embodiments, the determination module 220 may be configured to determine a composite weight sequence based on a tissue movement speed of at least one pixel point.

In some embodiments, the determination module 220 may be further configured to determine a speed level of the tissue movement speed of at least one pixel point; and determine a composite weight sequence based on the speed level.

In some embodiments, the determination module 220 may be further configured to divide, based on plane wave data in the imaging region, the imaging region into at least one sub-region and determine the speed level of the at least one sub-region; and determine the speed level of the sub-region where the at least one pixel point is located as the speed level of the tissue movement speed of the at least one pixel point.

In some embodiments, the determination module 220 may be further configured to determine a frequency shift of each of the at least one pixel point in the imaging region based on the plane wave data in the imaging region; preset at least one frequency shift threshold; and divide, based on the at least one frequency shift threshold and a proportional relationship between the frequency shift and the tissue movement speed, the imaging region into the at least one sub-region.

In some embodiments, the determination module 220 may be further configured to, for each pixel point, obtain a plurality of pieces of plane wave data via a plurality of emissions, and determine a plurality of initial frequency shifts based on the Doppler theorem, and obtain an average value of the plurality of initial frequency shifts and determine the average value as the frequency shift of the pixel point.

In some embodiments, the composite weight sequence may be determined based on Gaussian distributions, and the determination module 220 may determine a variance of at least one of the Gaussian distributions based on the speed level of the at least one sub-region, generate the composite weight sequence of the at least one sub-region based on the variance of the at least one Gaussian distribution, and determine the composite weight sequence of the at least one pixel point according to the sub-region where the at least one pixel point is located.

In some embodiments, the variance of the Gaussian distribution may be positively correlated with the speed level.

In some embodiments, the determination module 220 may be further configured to obtain a plurality of reference ultrasound images by emitting a focused wave targeted at the imaging region, determine tissue movement information between adjacent reference ultrasound images based on a correspondence between the adjacent reference ultrasound images, divide the imaging region into at least one sub-region based on the tissue movement information and determine the speed level of at least one sub-region, and determine the speed level of the tissue movement speed of the at least one pixel point based on the sub-region, among the at least one sub-region, where the at least one pixel point is located.

In some embodiments, the generation module 230 may be configured to determine, based on the beamforming result sequence and the composite weight sequence, the composite result to generate the ultrasound image of the imaging region.

In some embodiments, the generation module 230 may be further configured to determine a target beamforming result based on the beamforming result sequence, determine a target weight in the composite weight sequence based on a Gaussian distribution, determine a weight allocation result by allocating the target weight and weights on two sides of the target weight to the target beamforming result and beamforming results on two sides of the target beamforming result, respectively, and determine a composite result by compositing the beamforming result sequence based on the weight allocation result, and generate the ultrasound image based on the composite result.

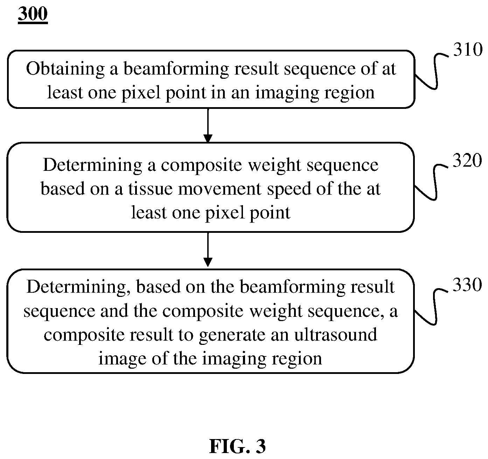

More descriptions regarding the acquisition module 210, the determination module 220 and the generation module 230 may be found in FIGs. 3-12 and their related descriptions.

It should be noted that the above description of the system for ultrasonic composite imaging 200 and its modules is only for convenience of description and cannot limit the present disclosure to the scope of the embodiments. It can be understood that for those skilled in the art, after understanding the principle of the system, it is possible to arbitrarily combine the modules or form a subsystem to connect with other modules without deviating from this principle. For example, in some embodiments, the acquisition module 210, the determination module 220, and the generation module 230 disclosed in FIG. 2 may be different modules in a system, or a module may realize the functions of two or more modules mentioned above. For example, each module may share a storage module, or each module may have its storage module. Such variations are all within the scope of protection of the present disclosure.

FIG. 3 is a flowchart illustrating an exemplary process for ultrasonic composite imaging according to some embodiments of the present disclosure. As shown in FIG. 3, process 300 includes the following operations. In some embodiments, one or more operations of process 300 shown in FIG. 3 may be implemented in the application scenario 100 of the system for ultrasonic composite imaging shown in FIG. 1. For example, process 300 shown in FIG. 3 may be stored in a storage device (e.g., the storage device 130) in the form of instructions, and called and /or executed by a processing device (e.g., the processing device 120).

In 310, a beamforming result sequence of at least one pixel point in an imaging region is obtained. In some embodiments, operation 310 may be performed by the acquisition module 210.

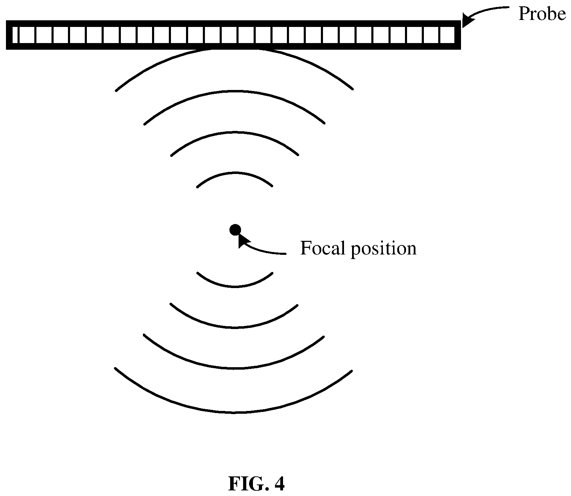

The imaging region refers to a region where ultrasonic composite imaging is required. In some embodiments, to observe the situation of a certain region, the processing device may perform the imaging through ultrasonic imaging, and the region may be referred to as the imaging region. Ultrasonic imaging modes may include ultrasonic B-mode imaging, and the ultrasonic B-mode imaging may be performed based on the focused wave shown in FIG. 4.

The pixel point refers to the smallest unit in the imaging region. The pixel point may include a pixel point in a two-dimensional scanning image or a voxel point in a three-dimensional scanning image. In some embodiments, the imaging region may include at least one pixel point.

The beamforming result refers to, for each pixel point in the imaging region, a beamforming result obtained by superimposing echo signals generated by the reflection of sound waves emitted by different emission array elements at the pixel point. The superposition manner may include delayed superposition.

The beamforming result sequence refers to a sequence of beamforming results of a pixel point, which is obtained by, for each pixel point, obtaining a beamforming result in each emission of multiple emissions, and arranging multiple beamforming results according to an emission order of multiple emissions. In some embodiments, each pixel point in the imaging region corresponds to a beamforming result sequence. The emission order refers to the sequence in which the sound waves are emitted one after another.

In some embodiments, the processing device may emit a plane wave targeted at the imaging region for multiple times. For each pixel point, the processing device may determine at least one beamforming result based on the plane wave of at least one emission of the plane wave and generate a beamforming result sequence.

In some embodiments, the processing device may emit a focused wave targeted at the imaging region for multiple times. For each pixel point, the processing device may determine at least one beamforming result based on the at least one emission of the focused wave and generate a beamforming result sequence of the pixel point based on the at least one beamforming result.

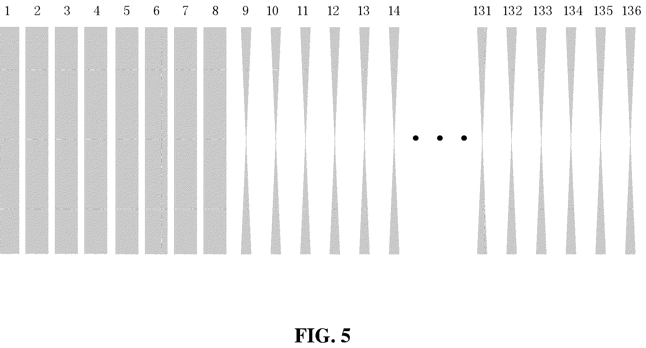

The focused wave is a sound wave that may be emitted by a plurality of array elements of a probe in cooperation with each other. The focused wave may focus on a focus point after propagating forward for a certain distance. After focusing, the sound wave may diverge again when it continues to propagate forward. The focus point may be referred to as a focal position, as shown in FIG. 4. In one embodiment, an emission corresponding to the beamforming result is a focused wave emission, and the focal positions of the focused wave emissions in adjacent emission orders are adjacent. For example, as shown in FIG. 5, the focal position of the focused wave 9 is adjacent to the focal position of the focused wave 10. Therefore, the emission order of the focused wave emission of the focused wave 9 is adjacent to the emission order of the focused wave emission of the focused wave 10.

In some embodiments, a portion of array elements in a probe of an ultrasound device (e.g., the ultrasound device 110) is used to emit the focused wave (e.g., the entire probe has 128 array elements, and 46 array elements are used). The array elements used have a sequence when emitting sound waves. For example, the array elements on two sides emit sound waves first, the array elements in the middle emit sound waves later, and the sound waves form a focused wave sound field through electronic delayed focusing.

In some embodiments, the processing device may adjust the focal position by adjusting a coordination relationship between emissions of the plurality of array elements. In some embodiments, when the ultrasound imaging is performed based on the focused wave, after the focused wave at a certain focal position is emitted, it may be regarded as completing one emission, that is, after the focused waves at two focal positions are emitted, it may be regarded as completing two focused wave emissions. As shown in the imaging sequence of FIG. 5, where numbers 9 to 136 are focused wave sequences, and since the focal position of the focused wave 9 is different from the focal position of the focused wave 10, the emissions of the focused wave 9 and the focused wave 10 may be regarded as two focused wave emissions.

In some embodiments, the ultrasound device needs to emit the focused wave for multiple times to generate a complete ultrasound image. For example, focused waves numbered 9 to 136 shown in FIG. 5 are emitted, that is, 128 focused wave emissions are performed. When the multiple focused wave emissions are performed, after one focused wave emission is completed, the focus position of the focused wave is changed and the next focused wave emission is performed.

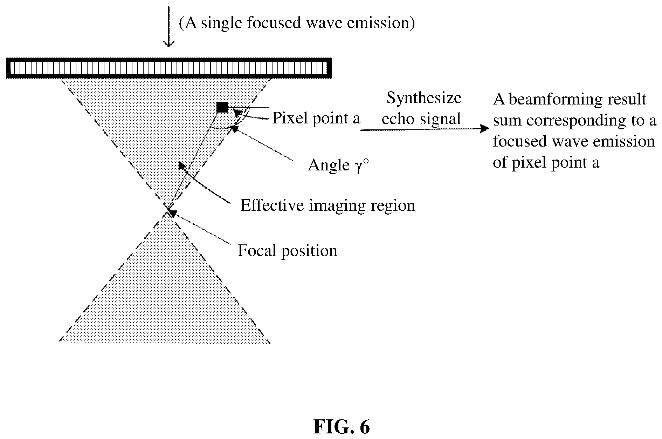

In a focused wave emission, the region covered by the sound wave from divergence to focusing and then to emission may be referred to as an effective imaging region of the focused wave emission, as shown in FIG. 6.

In some embodiments, when the effective imaging regions of the multiple focused wave emissions cover the imaging region, the multiple focused wave emissions may be regarded as focused wave emissions targeted at the imaging region.

In some embodiments, when a focused wave is emitted, if a certain pixel point in the imaging region is covered by the effective imaging region of the focused wave emission, the processing device may synthesize the echo signal of the pixel point to obtain the beamforming result of the pixel point corresponding to the focused wave emission. As shown in FIG. 6, the pixel point a in the imaging region falls within the effective imaging region of the focused wave emission. At this time, the processing device may synthesize the echo signal received by the corresponding array elements of the probe to obtain a beamforming result of the pixel point a corresponding to the focused wave emission. The beamforming result is recorded as sum.

In some embodiments, if multiple focused wave emissions are performed and the pixel point a falls within the effective imaging regions of the multiple focused wave emissions, a plurality of beamforming results of the pixel point a may be obtained.

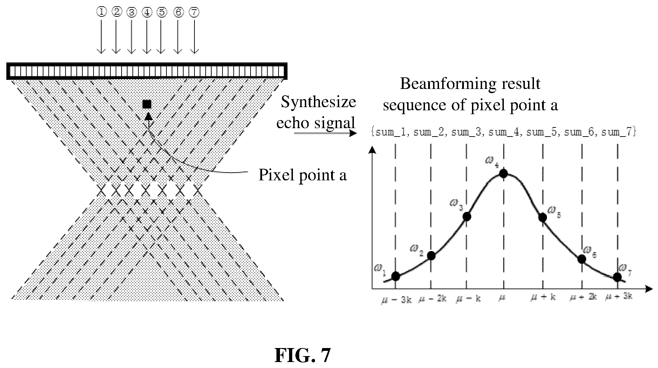

In some embodiments, the processing device may arrange multiple beamforming results of the same pixel point corresponding to the emission order, and a beamforming result sequence may be obtained. As shown in FIG. 7, the pixel point a of the imaging region falls within the effective imaging regions of the focused wave emissions ① to ⑦, so 7 beamforming results of the pixel point a may be obtained, which are sum_1, sum_2, sum_3, sum_4, sum_5, sum_6, and sum_7. Sum_1 corresponds to the focused wave emission ①, sum_2 corresponds to the focused wave emission ②, ..., sum_n corresponds to the nth (n is an integer greater than or equal to 1) focused wave emission. According to the focused wave emission order corresponding to the beamforming results, the above 7 beamforming results of the pixel point a may be sorted to obtain a beamforming result sequence of {sum_1, sum_2, sum_3, sum_4, sum_5, sum_6, sum_7}.

In some embodiments of the present disclosure, data for basic ultrasonic imaging may be obtained by obtaining a beamforming result sequence for each pixel point by emitting the focused wave targeted at the imaging region.

In 320, a composite weight sequence may be determined based on a tissue movement speed of at least one pixel. In some embodiments, operation 320 may be performed by the determination module 220.

The tissue movement speed refers to the movement speed of the tissue corresponding to a certain pixel point. The tissue refers to the voxels that make up the human body (e.g., a part of the human body, such as the heart, lungs, stomach, abdomen, blood vessels, bones (such as hip joints), etc.). For example, the tissue movement speed of the pixel point a may be the movement speed of the voxel of the human body corresponding to the pixel point a. In some embodiments, the tissue movement speed may be characterized by the frequency shift or displacement of the pixel point.

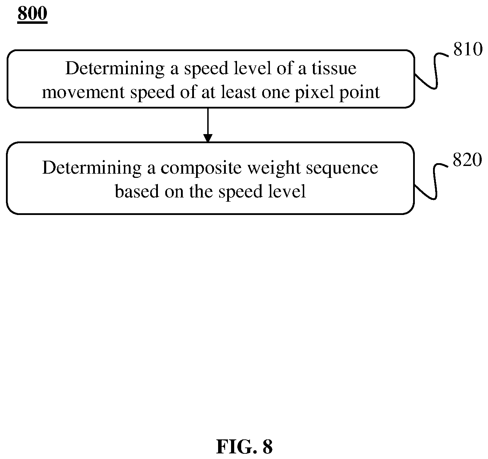

In some embodiments, the processing device may determine the tissue movement speed based on the Doppler effect generated by the tissue movement. More descriptions regarding obtaining the tissue movement speed may be found in FIG. 8 and its related description.

The composite weight refers to the coherent composite coefficient corresponding to each beamforming result in the beamforming result sequence of each pixel point when the coherent composite is performed.

The composite weight sequence refers to a sequence composed of coherent composite coefficients corresponding one-to-one to the elements in the beamforming result sequence. The count of elements in the composite weight sequence is the same as the count of elements in the beamforming result sequence, which is a one-to-one correspondence. For example, as shown in FIG. 7, the composite weight sequence corresponding to the beamforming result sequence {sum_1, sum_2, sum_3, sum_4, sum_5, sum_6, sum_7} may be $\{a_1, a_2, a_3, a_4, a_5, a_6, a_7\}$.

In some embodiments, the processing device may determine the composite weight sequence based on the speed level of the tissue movement speed of the at least one pixel. More descriptions regarding the speed level and the process of determining the composite weight sequence based on the speed level may be found in FIG. 8 and its related description.

In 330, based on the beamforming result sequence and the composite weight sequence, a composite result is determined to generate the ultrasound image of the imaging region. In some embodiments, operation 330 may be performed by the generation module 230.

The composite result refers to a coherent composite result of each pixel point.

In some embodiments, the processing device may directly multiply each of all beamforming results of each pixel point by the corresponding composite weight and then add all the products to obtain the composite result of each pixel point. Further, the processing device may reconstruct the composite result of at least one pixel point in the imaging region to obtain the ultrasound image of the imaging region. For example, as shown in FIG. 7, the composite result of pixel point a may be:

$$\text{sum\_1} \times a_1 + \text{sum\_2} \times a_2 + \text{sum\_3} \times a_3 + \text{sum\_4} \times a_4 + \text{sum\_5} \times a_5 + \text{sum\_6} \times a_6 + \text{sum\_7} \times a_7.$$

In some embodiments, for each pixel point in the imaging region, the processing device may select N (N is an integer less than the count of all beamforming results of the pixel point) beamforming results from the beamforming result sequence of the pixel point and determine N composite weights corresponding to the N beamforming results. Further, the processing device may multiply each of the N beamforming results of each pixel point by the corresponding composite weight and add all the products to obtain the composite result of the pixel point, thereby obtaining the ultrasound image of the imaging region. N may be determined based on the imaging effect. For example, as shown in FIG. 7, the processing device may select sum_3, sum_4, sum_5, and the corresponding $a_3$, $a_4$, $a_5$, and the composite result of the pixel point a may be: $\text{sum\_3} \times a_3 + \text{sum\_4} \times a_4 + \text{sum\_5} \times a_5$.

In some embodiments, for each pixel point in the imaging region, when multiple emissions are performed, the intensity of the echo signal reflected at the pixel point is different for different emissions. The processing device may determine the count of echo signals exceeding an intensity threshold as N, and perform coherent composition based on the beamforming results corresponding to the N emissions. The intensity threshold may be set manually.

In some embodiments, for regions with different levels of tissue movement speed, the N corresponding to the pixel point may be different. For example, in the high-speed region, the tissue moves very fast, and the correlation between adjacent emissions is very low. It is meaningless to take N too large, that is, N may be relatively small. In other words, only the data of the most relevant several emissions among the adjacent emissions emitted perpendicular to the pixel point are taken for coherent composition.

In some embodiments, in a beamforming result sequence for a certain pixel point, the quality of the beamforming result corresponding to the emission perpendicular to the pixel point is the best. Based on an angular relationship between other emissions and the pixel point, an angle interval (e.g., [80°, 100°]) may be set, and the beamforming results of N emissions within the angle interval may be used for the coherent composition of the pixel point. The angle refers to an angle between a line connecting a focus and a certain pixel point, and a straight line parallel to the probe. For example, as shown in FIG. 6, the angle between the emission and the pixel point a is $\gamma°$. As another example, as shown in FIG. 7, the angle between the fourth focused wave emission and the pixel point a is 90°.

In some embodiments of the present disclosure, by selecting some elements in the beamforming result sequence and the composite weight sequence to determine the composite result and generate the ultrasound image of the imaging region, better results may be obtained while reducing the amount of calculation.

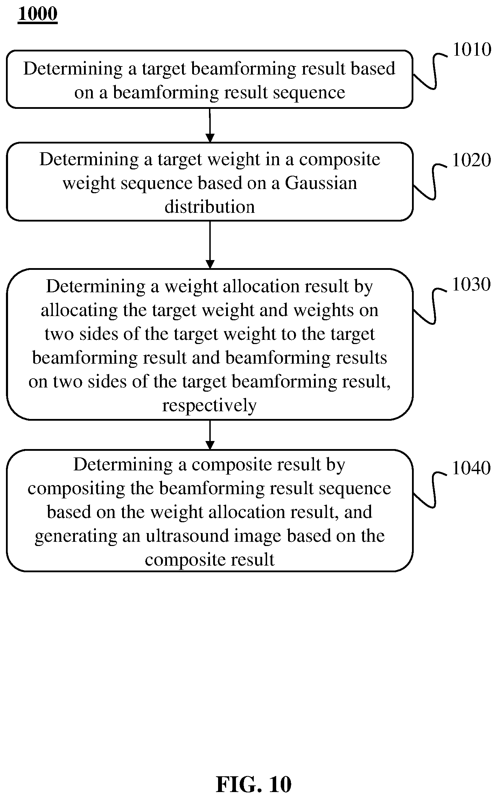

In some embodiments, the processing device may composite the beamforming result sequence based on a weight distribution result, determine the composite result, and generate the ultrasound image. More descriptions regarding the weight distribution result and how to composite the beamforming results based on the weight distribution result may be found in FIG. 10 and its related description.

In some embodiments of the present disclosure, a composite result is determined based on the beamforming result sequence and the composite weight sequence determined based on the tissue movement speed of at least one pixel point, and then the ultrasound image of the imaging region is generated. The movement detection technology and the composite imaging technology may be combined to improve the resolution of the ultrasound image by using the composite imaging technology, while ensuring the resolution of imaging of moving tissue regions such as the heart.

It should be noted that the above description of process 300 is only for example and explanation and does not limit the scope of application of the present disclosure. For those skilled in the art, various modifications and changes may be made to process 300 under the guidance of the present disclosure. However, these modifications and changes are still within the scope of the present disclosure. For example, operation 310 and operation 320 may be performed simultaneously.

FIG. 8 is a flowchart illustrating an exemplary process for determining a composite weight sequence according to some embodiments of the present disclosure. As shown in FIG. 8, process 800 includes the following operations. In some embodiments, one or more operations of process 800 shown in FIG. 8 may be implemented in the application scenario 100 of the system for ultrasonic composite imaging shown in FIG. 1. For example, process 800 shown in FIG. 8 may be stored in a storage device (e.g., the storage device 130) in the form of instructions, and called and/or executed by a processing device (e.g., the processing device 120).

In 810, a speed level of a tissue movement speed of at least one pixel point is determined.

The speed level refers to words, numbers or letters that may reflect the tissue movement speed. For example, the speed levels may include high speed, medium speed, and low speed. Each speed level has a corresponding variance and a corresponding composite weight sequence. In some embodiments, if the speed level to which the tissue movement speeds at two pixel points belong is the same, then the two tissue movement speeds may share the composite weight sequence corresponding to the speed level.

In some embodiments, the processing device may divide, based on plane wave data in the imaging region, the imaging region into at least one sub-region and determine a speed level of the at least one sub-region, and determine the speed level of the sub-region where the at least one pixel point is located as the speed level of the tissue movement speed of the at least one pixel point.

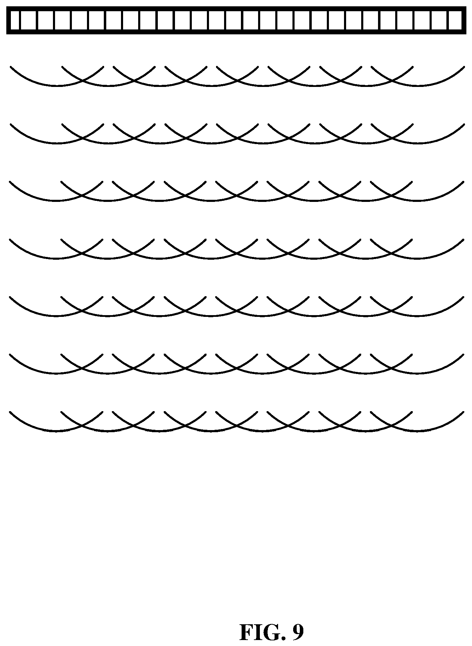

As shown in FIG. 9, the plane wave refers to a wave whose wavefronts are parallel to each other. Compared with the focused wave, a plane wave emission does not have a focusing step. The plane wave data refers to the echo data of the plane wave at each pixel point in the imaging region.

In some embodiments, the processing device may emit sound waves through all the array elements of the probe at the same time, and plane wave data may be obtained based on the echo data of each pixel point in the imaging region. As shown in the imaging sequence of FIG. 5, numbers 1 to 8 are plane wave sequences, and each vertical line in the figure represents one emission.

The sub-region refers to a region divided from the imaging region. All sub-regions may form the imaging region.

In some embodiments, the processing device may determine the frequency shift of each of the at least one pixel in the imaging region based on the plane wave data in the imaging region in a preset manner and preset at least one frequency shift threshold. Based on the at least one frequency shift threshold and a proportional relationship between the frequency shift and the tissue movement speed, the imaging region may be divided into at least one sub-region, and the speed level of at least one sub-region may be determined.

The preset manner refers to a manner for determining the frequency shift of the pixel point. In some embodiments, the preset manner may include but is not limited to the Doppler theorem.

The frequency shift refers to a difference between emitted and received frequencies due to the Doppler effect.

In some embodiments, for each pixel, the processing device may obtain a plurality of pieces of plane wave data via a plurality of emissions, determine a plurality of initial frequency shifts based on the Doppler theorem, and determine an average value of the plurality of initial frequency shifts as the frequency shift of the pixel point. In each emission of the plane wave, a set of plane waves may be obtained, and a relatively accurate frequency shift may be determined based on 5 to 15 emissions.

The Doppler theorem is as follows. The wavelength of a plane wave changes due to the relative movement between the imaging region and the transducer. If the imaging region moves toward the transducer, the wavelength is compressed and becomes shorter, and the frequency of the plane wave becomes higher. If the imaging region moves away from the transducer, the opposite effect occurs, the wavelength becomes longer, and the frequency of the plane wave becomes lower.

In some embodiments, by emitting the plane wave the plurality of times to obtain the plurality of pieces of plane wave data, the processing device determines the plurality of initial frequency shifts of the same pixel point based on the Doppler theorem, calculates the frequency shift average value of the plurality of initial frequency shifts, and determines the frequency shift average value as the frequency shift of the pixel point.

In some embodiments, the processing device may determine the difference between the frequency of the emission and the frequency of the echo signal at each pixel as the frequency shift of the pixel point.

In some embodiments of the present disclosure, the plurality of initial frequency shifts are calculated based on the Doppler effect generated by the movement of the imaging region, and the average value of the plurality of initial frequency shifts is calculated, which may improve the accuracy of calculating the tissue movement speed and help determine regions with fast tissue movement speed such as the myocardium.

The frequency shift threshold refers to a threshold condition that the frequency shifts corresponding to different sub-regions in the at least one sub-region need to meet and, which may be preset based on relevant knowledge or historical experience. In some embodiments, the faster the tissue movement speed in the imaging region, the smaller the interval between the plurality of frequency shift thresholds.

In some embodiments, since the frequency shifts of adjacent pixels should be continuous, the processing device may determine a frequency shift threshold interval where the frequency shift of at least one pixel point in the imaging region is located based on the frequency shift threshold, and determine the region where at least one pixel point in the same frequency shift threshold interval is located as the same sub-region. The frequency shift threshold interval refers to the numerical interval between two adjacent frequency shift thresholds. In some embodiments, the processing device may define one or more sub-regions as a high-speed movement region, a medium-speed movement region, a low-speed movement region, etc., based on the frequency shift threshold interval corresponding to the sub-region and the proportional relationship between the frequency shift and the tissue movement speed. In some embodiments, in order to make the signal changes between adjacent pixel points more uniform, the processing device may further divide the divided high-speed movement region, medium-speed movement region, and low-speed movement region into more speed regions. The count of movement region divisions of the imaging region with a faster movement speed may be greater than the count of movement region divisions of the imaging region with a slower movement speed, and the specific count is positively correlated with the movement speed. For example, if the movement speed of the heart tissue is faster, the count of movement region divisions may be higher; if the movement speed of the stomach tissue is slower, the count of movement region divisions may be lower.

In some embodiments of the present disclosure, the imaging region is divided into at least one sub-region based on the frequency shift threshold and the relationship between the frequency shift and the tissue movement speed. When the tissue movement speed is uncertain, the sub-region may be divided and the corresponding speed level may be determined.

In some embodiments, the processing device may determine the tissue movement speed of at least one pixel point in the imaging region based on the plane wave data in the imaging region in a preset manner and preset at least one speed threshold. Further, based on the at least one speed threshold, the imaging region may be divided into at least one sub-region and the speed level of the at least one sub-region may be determined.

In some embodiments, by emitting the plane wave a plurality of times to obtain a plurality of pieces of plane wave data, the processing device may determine a plurality of initial frequency shifts of the same pixel point based on the Doppler theorem and determine the tissue movement speed based on the plurality of initial frequency shifts and the Doppler velocity measurement equation.

In some embodiments, the processing device may determine the tissue movement speed based on the average value of the plurality of initial frequency shifts of the pixel point.

In some embodiments, the processing device may also determine the tissue movement speed based on historical plane wave data and current plane wave data.

In some embodiments, the processing device may determine the tissue movement speed by vector matching. In some embodiments, the processing device may obtain the current plane wave data based on the plane wave, construct a first eigenvector based on the current plane wave data, search in a plane wave database based on the first eigenvector, determine a first reference vector whose similarity with the first eigenvector meets a preset condition, and determine a reference tissue movement speed corresponding to the first reference vector as the current tissue movement speed. The plane wave database stores the first reference vector constructed based on the historical plane wave data and the corresponding reference tissue movement speed.

In some embodiments, the processing device may also determine the tissue movement speed by machine learning. For example, the processing device may determine the tissue movement speed based on a speed model.

In some embodiments, the speed model may be a machine learning model, such as a neural network model (NN), a deep neural network model (DNN), etc., or any combination thereof. In some embodiments, by inputting the plane wave data into the speed model, the tissue movement speed may be output.

In some embodiments, the processing device may obtain training sample data of plane wave data and its tissue movement speed label data, input the training sample data of plane wave data into an initial speed model and output calculation result data. The processing device may further update model parameters of the initial speed model based on the calculation result data and tissue movement speed label data, and continue training until the desired model, i.e., the speed model, is obtained.

In some embodiments, the training sample data of plane wave data and tissue movement speed label data may be obtained from historical data of tissue movement detection based on the plane wave. For example, the processing device may obtain the training sample data of plane wave data and tissue movement speed label data from ultrasound imaging historical record data accumulated based on movement detection scenes of the imaging region by the ultrasound device.

In some embodiments, the process of the processing device dividing the imaging region into at least one sub-region based on the tissue movement speed of at least one pixel point and determining the speed level of at least one sub-region is similar to the process of the processing device dividing the imaging region into at least one sub-region based on the frequency shift of at least one pixel point and determining the speed level of at least one sub-region, which is not be repeated here.

In some embodiments, the processing device may determine the speed level of the sub-region corresponding to the pixel point as the speed level of the tissue movement speed of the pixel point.

In some embodiments of the present disclosure, movement detection is performed on the imaging region by emitting the plane wave to obtain the tissue movement speed of at least one pixel point in the imaging region, and then the sub-region is divided and the speed level of the sub-region is determined. The speed level of the tissue movement speed of the pixel point may be determined quickly and effectively, which improves the composition efficiency.

In 820, a composite weight sequence is determined based on the speed level.

In some embodiments, for sub-regions of different speed levels within the imaging region, the processing device may set a composite coefficient of the emission time dimension to determine a composite weight sequence corresponding to the sub-regions of different speed levels.

In some embodiments, the composite weight sequence may be determined based on Gaussian distributions.

In some embodiments, the processing device may determine a variance of at least one of the Gaussian distributions based on the speed level of at least one sub-region, generate a composite weight sequence of at least one sub-region based on the variance of the at least one Gaussian distribution, and determine the composite weight sequence of at least one pixel point based on a sub-region where at least one pixel point is located.

The Gaussian distribution is a continuous probability distribution, also referred to as a normal distribution. The variance of Gaussian distribution represents the variance of the vertical axis, which is used to measure the degree of deviation between the value of the vertical axis and the average value of the vertical axis. The smaller the value $\sigma^2$, the smaller the fluctuation of the value of the vertical axis, and the lower or wider the image; the larger the value $\sigma^2$, the greater the fluctuation of the value of the vertical axis, and the higher or narrower the image.

In some embodiments, for sub-regions of different speed levels, based on the speed level, the variance of the Gaussian distribution suitable for the speed level may be manually set, or it may be a system default setting.

In some embodiments, the processing device may determine the variance of the Gaussian distribution based on angle differences between a plurality of focused wave emissions and the pixel point. The angle difference refers to the difference between the angle of the emission that is not perpendicular to the pixel point and the angle of the emission that is perpendicular to the pixel point to the pixel point (that is, the angle between the emission that is not perpendicular to the pixel point and the pixel point minus 90 °). The larger the angle difference, the worse the correlation between the two pieces of adjacent emission data, and the larger the variance of the Gaussian distribution. More descriptions regarding the angle may be found in FIG. 3 and its related description.

In some embodiments, the processing device may also determine the variance of the Gaussian distribution based on the speed level and the angle difference. For example, the processing device may evaluate the speed level and the angle difference respectively, determine evaluation values corresponding to the speed level and the angle difference, perform weighted summation based on the evaluation values, and determine the variance based on the summation result. In the weighted summation, the weight of the evaluation value of the speed level is greater than the weight of the evaluation value of the angle difference.

In some embodiments, the variance of the Gaussian distribution is positively correlated with the speed level.

In some embodiments, the distribution of the composite weight sequence may be set in the manner of Gaussian distribution.

In some embodiments, when the speed levels of a plurality of sub-regions are different, based on the proportional relationship between the speed level and the variance of the Gaussian distribution, the processing device may determine the variance of the Gaussian distribution corresponding to the speed level of each sub-region, and determine the corresponding composite weight sequence. Further, according to the sub-region where the at least one pixel point is located, the composite weight sequence of at least one pixel point is determined. The higher the speed level of the sub-region, that is, the higher the tissue movement speed, the greater the variance of the Gaussian distribution of the corresponding composite weight sequence, and the more dispersed the magnitudes of the weights in the composite weight sequence.

In some embodiments, the processing device may preset the variance based on the fact that the variance of the composite weight sequence is positively correlated with the speed level and determine the composite weight sequence. For example, the processing device may set the variance $\sigma^2$ based on the speed level, and the higher the speed level, the larger the set variance $\sigma^2$. The processing device may also set an expected value $\mu$, and form a Gaussian distribution curve according to the set variance $\sigma^2$ and the expected value $\mu$. The Gaussian distribution curve is continuous. The processing device may discretize the Gaussian distribution curve to obtain a discrete composite weight sequence. The discretization processing may include selecting, on the horizontal axis of the Gaussian distribution curve, an interval centered on the expected value $\mu$ or its vicinity, dividing the length of the interval by N ( N is the count of beamforming results to be composited) and obtaining a division result k; taking a weight at or near the expected value $\mu$, and taking a weight for every interval k, including taking a weight at or near $\mu$ - k, taking a weight at or near $\mu$ + k, taking a weight at or near $\mu$ - 2k, taking a weight at or near $\mu$ + 2k, and so on, thereby obtaining the composite weight sequence.

In order to improve the composition efficiency, the composition effect may be slightly compromised. At this time, the level of the tissue movement speed may be divided to obtain a plurality of speed levels, such as high speed, a medium speed, and a low speed. Each speed level has a corresponding variance and a corresponding composite weight sequence. In this case, if the speed level of the tissue movement speed at two pixel points is the same, the two tissue movement speeds may share the composite weight sequence corresponding to the speed level.

In some embodiments, the processing device may determine the speed level to which the tissue movement speed at the pixel point belongs based on the tissue movement speed at the pixel point, and obtain the corresponding composite weight sequence based on the proportional relationship between the speed level and the variance of the Gaussian distribution sequence, and the speed level to which the tissue movement speed at the pixel point belongs. For example, as shown in FIG. 7, the processing device may obtain the composite weight sequence as $\{\omega_1, \omega_2, \omega_3, \omega_4, \omega_5, \omega_6, \omega_7\}$ based on the tissue movement speed at the pixel point a, and the higher the tissue movement speed at the pixel point a, the greater the variance of the composite weight sequence.

In some embodiments of the present disclosure, the variance of the Gaussian distribution may be roughly determined based on the speed level. The higher the speed level, the worse the correlation between two adjacent pieces of emission data, and the larger the variance of the Gaussian distribution. The lower the speed level, the better the correlation between two adjacent emission data, and the smaller the variance of the Gaussian distribution. This helps to determine a more reasonable composite weight sequence and reduce the impact of emission data with poor correlation on ultrasonic imaging.

In some embodiments of the present disclosure, the composite weight sequence is determined based on the speed level of the tissue movement speed, which improves the image resolution of ultrasound imaging by using coherent composite technology while avoiding the influence of tissue movement.

It should be noted that the above description of process 800 is only for example and illustration and does not limit the scope of application of the present disclosure. For those skilled in the art, various modifications and changes may be made to process 800 under the guidance of the present disclosure. However, these modifications and changes are still within the scope of the present disclosure.

In some embodiments, when the variance of the Gaussian distribution does not take into account the angle difference of the pixel point, the composite weight sequence is not only related to the tissue movement speed, but also to the angle differences between the plurality of focused wave emissions and the pixel point. Since the beamforming result of the emission perpendicular to the pixel point has the best imaging effect, the influence of the angle may be further considered on the basis of the composite weight sequence determined based on the tissue movement speed. For example, the processing device may multiply each composite weight in the composite weight sequence by a corresponding emission angle coefficient, and the emission angle coefficient may be preset based on the angle difference. The larger the angle difference, the smaller the angle coefficient.

FIG. 10 is a flowchart illustrating an exemplary process for generating an ultrasound image of an imaging region according to some embodiments of the present disclosure. As shown in FIG. 10, process 1000 includes the following operations. In some embodiments, one or more operations of process 1000 shown in FIG. 10 may be implemented in the application scenario 100 of the system for ultrasonic composite imaging shown in FIG. 1. For example, process 1000 shown in FIG. 10 may be stored in a storage device (e.g., the storage device 130) in the form of instructions, and called and /or executed by a processing device (e.g., the processing device 120).

In 1010, a target beamforming result is determined based on the beamforming result sequence.

The target beamforming result refers to the beamforming result of a beam in which the focused wave is emitted perpendicularly to the pixel point or approximately perpendicularly to the pixel point.

In some embodiments, the processing device may determine a beamforming result that satisfies a first preset condition in the beamforming result sequence as the target beamforming result.

The first preset condition refers to a condition for determining whether a beamforming result may be used as the target beamforming result. In some embodiments, the first preset condition may include a focused wave corresponding to the beamforming result of a pixel point being emitted perpendicularly to the pixel point or approximately perpendicularly to the pixel point, that is, a target emission. In some embodiments, the beamforming result that satisfies the first preset condition in the beamforming result sequence may include a beamforming result corresponding to an emission perpendicular to at least one pixel point in the beamforming result sequence.

In some embodiments, as shown in FIG. 7, pixel point a in the imaging region falls within the effective imaging region of focused wave emissions ① to ⑦. In some embodiments, in the focused wave emissions ① to ⑦, if pixel point a is in the emission center region of the focused wave emission ④, the focused wave emission ④ may be referred to as an emission perpendicular to the pixel point a. Therefore, the emission perpendicular to the pixel point in a plurality of emissions is referred to as the target emission.

In some embodiments, in the focused wave emissions ① to ⑦, pixel point a is not in the emission center region of any focused wave emission, that is, in the plurality of emissions, there is no emission perpendicular to the pixel point a. In this case, which focused wave emission center region the pixel point a is close to may be determined, and this focused wave emission is referred to as the emission approximately perpendicular to the pixel point a and is designated as the target emission.

In some embodiments, the processing device may use the beamforming result corresponding to the focused wave emission perpendicular to the pixel point or approximately perpendicular to the pixel point in multiple emissions as the target beamforming result, that is, the beamforming result corresponding to the target emission may be used to determine the target beamforming result. For example, as shown in FIG.7, the processing device may determine the beamforming result sum_4 corresponding to the fourth focused wave emission as the target beamforming result.

In 1020, a target weight in the composite weight sequence is determined based on a Gaussian distribution.

The target weight refers to the weight at the expected value or the weight near the expected value in the Gaussian distribution sequence.

In some embodiments, the processing device may determine the composite weight that satisfies a second preset condition as the target weight.

The second preset condition refers to a condition for determining whether a composite weight in the composite weight sequence may be used as the target weight. In some embodiments, the second preset condition may include the composite weight being a weight at an expected value or a weight near an expected value in the composite weight sequence.

In some embodiments, the processing device may determine the composite weight at the expected value $\mu$ in the composite weight sequence or the composite weight near the expected value $\mu$ as the target weight. For example, the processing device may set the expected value $\mu \pm \varepsilon$ as a range near the expected value $\mu$, and any point of non-expected value in the range as a point near the expected value $\mu$, that is, the processing device may designate a composite weight at any point in $[\mu - \varepsilon, \mu + \varepsilon]$ as the target weight. For example, in the above composite weight sequence of $\{ \omega_1, \omega_2, \omega_3, \omega_4, \omega_5, \omega_6, \omega_7 \}$, if $\omega_4$ is a composite weight at a certain point in $[\mu - \varepsilon, \mu + \varepsilon]$, $\omega_4$ may be designated as the target weight.

In 1030, a weight allocation result is determined by allocating the target weight and weights on two sides of the target weight to the target beamforming result and beamforming results on two sides of the target beamforming result, respectively.

The weight allocation refers to allocating the target weight in the composite weight sequence of each pixel point to the target beamforming result and allocating the weights on two sides of the target weight to the beamforming results on two sides of the target beamforming result. The result obtained after weight allocation is the weight allocation result.

In some embodiments, the processing device may obtain a beamforming result sequence of the pixel point within the imaging region. The beamforming result sequence includes beamforming results that are arranged according to their respective emission orders. When compositing a plurality of beamforming results of the pixel point, a composite weight sequence is obtained based on the tissue movement speed or the corresponding speed level of the pixel point. The target beamforming result corresponds to a target sub-emission that is perpendicular to or approximately perpendicular to the pixel in a plurality of emissions, the target weight is the weight at the expected value in the composite weight sequence or the weight adjacent to the expected value, and the weights on two sides of the target weight gradually decrease. The target weight in the composite weight sequence is allocated to the target beamforming result, and the weights on two sides of the target weight are correspondingly allocated to the beamforming results on two sides of the target beamforming result, thereby obtaining a weight allocation result.

For example, as shown in FIG. 7, if the beamforming result sequence of pixel point a is {sum_1, sum_2, sum_3, sum_4, sum_5, sum_6, sum_7}, and the composite weight sequence is $\{ \omega_1, \omega_2, \omega_3, \omega_4, \omega_5, \omega_7 \}$, where the target beamforming result is sum_4 and the target weight is $\omega_4$, $\omega_4$ may be allocated to sum_4. $\omega_1, \omega_2, \omega_3$ on one side of $\omega_4$ may be allocated to sum_1, sum_2, and sum_3 on one side of sum_4, respectively, and $\omega_5, \omega_6$, and $\omega_7$ on the other side of $\omega_4$ may be allocated to sum_5, sum_6, and sum_7 on the other side of sum_4, respectively, such that allocation results $\omega_1 \times$sum_1, $\omega_2 \times$sum_2, $\omega_3 \times$sum_3, $\omega_4 \times$sum_4, $\omega_5 \times$sum_5, $\omega_6 \times$sum_6, and $\omega_7 \times$sum_7 may be obtained.

In 1040, a composite result is determined by compositing the beamforming result sequence based on the weight allocation result, and the ultrasound image is generated based on the composite result.

In some embodiments, the processing device may perform a weighted summation on the beamforming result sequence based on the weight allocation result, and use the weighted summation result as the composite result. For example, the composite result of pixel point a is: $\omega_1 \times$sum_1+$\omega_2 \times$sum_2+$\omega_3 \times$sum_3+ $\omega_4 \times$sum_4+ $\omega_5 \times$sum_5+ $\omega_6 \times$sum_6+$\omega_7 \times$sum_7.

In some embodiments, the processing device may reconstruct a composite result of at least one pixel point in the imaging region to generate the ultrasound image.

In some embodiments of the present disclosure, when the target weight is assigned to the target beamforming result and weights on two sides of the target weight are assigned to beamforming results on two sides of the target beamforming result, the proportion of beamforming results of emissions close to the target emission in the composition may be increased, and the proportion of beamforming results of emissions far from the target emission in the composition may be reduced, thereby avoiding interference caused by excessive movement of tissues such as the heart and ensuring the resolution of the ultrasound image.

It should be noted that the above description of process 1000 is only for example and explanation and does not limit the scope of application of the present disclosure. For those skilled in the art, various modifications and changes may be made to process 1000 under the guidance of the present disclosure. However, these modifications and changes are still within the scope of the present disclosure. For example, operation 1010 and operation 1020 may be performed simultaneously.

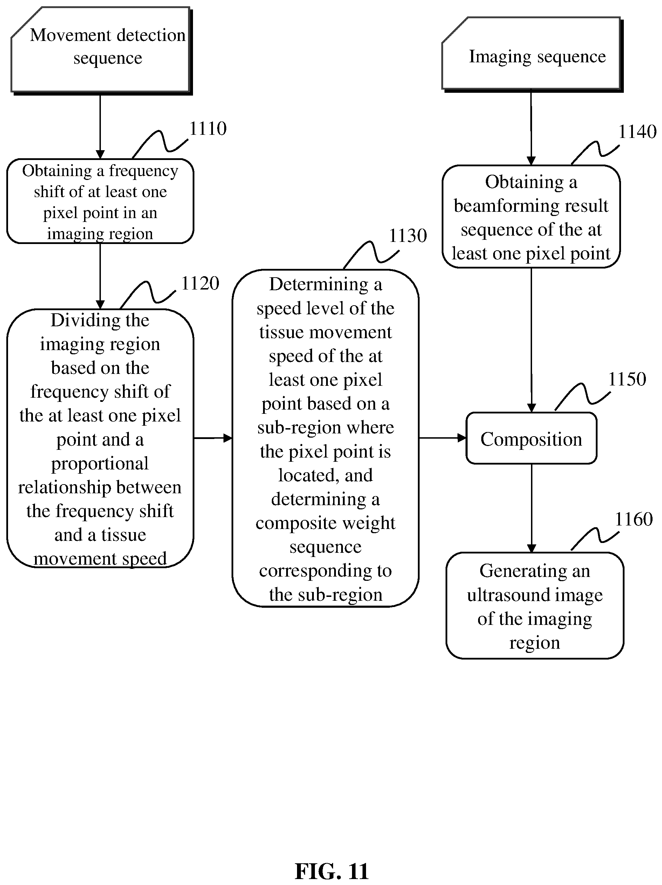

FIG. 11 is a flowchart illustrating an exemplary process for obtaining an ultrasound image based on an emission order according to some embodiments of the present disclosure. As shown in FIG. 11, process 1100 includes the following steps. In some embodiments, one or more operations of process 1100 shown in FIG. 11 may be implemented in the application scenario 100 of the system for ultrasonic composite imaging shown in FIG. 1. For example, process 1100 shown in FIG. 11 may be stored in a storage device (e.g., the storage device 130) in the form of instructions, and called and/or executed by a processing device (e.g., the processing device 120).

In some embodiments, the movement detection is performed in a plane wave manner, and a plurality of plane wave emissions are performed. The calculation error of the tissue movement speed is reduced by averaging. A movement detection sequence used may be numbered 1 to 8 as shown in FIG. 5, and 8 plane wave emissions are performed. In this case, to obtain a complete ultrasound image, the movement detection sequence and the imaging sequence are combined to obtain a complete emission sequence as shown in FIG. 5. The imaging sequence may be numbered 9 to 136 as shown in FIG. 5, and 128 focused wave emissions are performed.

In 1110, a frequency shift of each of at least one pixel point in an imaging region is determined.

In some embodiments, the processing device may obtain plane wave data based on the movement detection to obtain the frequency shift of each of at least one pixel point in the imaging region.

In 1120, the imaging region is divided based on the frequency shift of the at least one pixel point and a proportional relationship between the frequency shift and the tissue movement speed.

In some embodiments, the processing device may divide the imaging region based on the frequency shift of the at least one pixel point and the proportional relationship between the frequency shift and the tissue movement speed to obtain a plurality of sub-regions and speed levels corresponding to the plurality of sub-regions.

In 1130, the speed level of the tissue movement speed of the at least one pixel point is determined based on a sub-region where the pixel point is located, and a composite weight sequence corresponding to the sub-region is determined.

In 1140, a beamforming result sequence of the at least one pixel point is obtained.

In some embodiments, the processing device may obtain a beamforming result of a pixel point based on echo data of the pixel point.

In 1150, a composition is performed.

In some embodiments, the processing device may determine a composite result based on the beamforming result sequence and the composite weight sequence.

In 1160, an ultrasound image of the imaging region is generated.

In some embodiments, the processing device may generate the ultrasound image of the imaging region based on the composite result of the at least one pixel point.

The more times the plane wave is emitted for movement detection, the higher the accuracy of the frequency shift calculation. However, there is a certain limit for the emission times. After the count of the plane wave emissions reaches a certain limit, the influence of increasing the count of the plane wave emissions on the accuracy of the frequency shift calculation reduces. Therefore, when using movement detection to make a rough division of the imaging region, the accuracy required for the frequency shift calculation is not high, and the count of the plane wave emissions may be set at 5 to 15 times.

In some embodiments, the processing device may also perform movement detection based on an optical flow method of an image sequence, which calculates a tissue movement speed between adjacent frames of images based on the correlation between the adjacent frames and a correspondence between a current frame and a previous frame.

In some embodiments, the processing device may emit the focused wave for the imaging region to obtain a plurality of reference ultrasound images, determine tissue movement information between adjacent reference ultrasound images based on a correspondence between the adjacent reference ultrasound images, divide the imaging region into at least one sub-region based on the tissue movement information and determine the speed level of the at least one sub-region, and determine the speed level of the sub-region where the at least one pixel point is located as the speed level of the tissue movement speed of the at least one pixel point.

In some embodiments of the present disclosure, the plurality of reference ultrasound images may be obtained using the imaging sequence. For example, numbers 1 to 136 are all focused waves, and the processing device may designate the ultrasound images corresponding to the focused waves numbered 1 to 8 as the reference ultrasound images. Using the ultrasound image sequence for movement detection does not require adding a movement detection sequence to the emission sequence (i.e., there is no need to emit a plane wave for movement detection), which may reduce the storage burden of the hardware. The emission sequence may be used for both ultrasound B-mode imaging and movement detection. However, the first few frames of ultrasound images are required to perform movement detection to guide the ultrasound imaging coherent composite of the current frame, so the first few frames of images may have poor image resolution.

It should be noted that the above description of process 1100 is only for example and explanation and does not limit the scope of application of the present disclosure. For those skilled in the art, various modifications and changes may be made to process 1100 under the guidance of the present disclosure. However, these modifications and changes are still within the scope of the present disclosure.

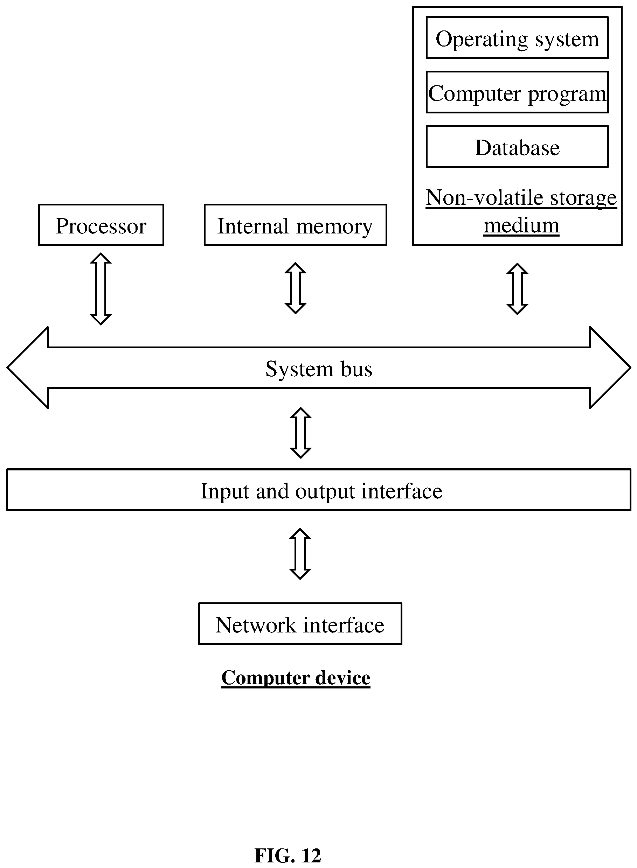

FIG. 12 is a schematic diagram illustrating an exemplary computer device according to some embodiments of the present disclosure.

As shown in FIG. 12, in some embodiments, a computer device may include a processor, a memory, and a network interface connected via a system bus. The processor of the computer device is configured to provide computing and control capabilities. The memory of the computer device includes a non-volatile storage medium and an internal memory. The non-volatile storage medium stores an operating system, a computer program, and a database. The internal memory provides an environment for the operation of the operating system and the computer program in the non-volatile storage medium. The database of the computer device is used to store ultrasound composite imaging data. The network interface of the computer device is used to communicate with an external terminal via a network connection. The computer device also includes an input and output interface, which is a connection circuit for exchanging information between the processor and the external device. The input and output interface is connected to the processor via a bus and is referred to as an I/O interface. When the computer program is executed by the processor, the method for ultrasonic composite imaging is implemented.

Those skilled in the art may understand that the structure shown in FIG. 12 is merely a block diagram of a partial structure related to the solution of the present application and does not constitute a limitation on the computer device to which the solution of the present application is applied. The specific computer device may include more or fewer components than shown in the figure, or combine certain components, or have a different arrangement of components.

One of the embodiments of the present disclosure also provides a device for ultrasonic composite imaging, which includes at least one memory and at least one processor. The at least one memory is used to store computer instructions, and the at least one processor is used to execute at least part of the computer instructions to implement the method for ultrasonic composite imaging described in the embodiment of the present disclosure.

One of the embodiments of the present disclosure further provides a non-transitory computer-readable storage medium storing computer instructions. When the computer instructions are executed by the processor, the method for ultrasonic composite imaging described in the embodiments of the present disclosure is implemented.

The beneficial effects of the present disclosure include but are not limited to the following. First, the composite result is determined based on the beamforming result sequence and the composite weight sequence determined based on the tissue movement speed of at least one pixel point, and then the ultrasound image of the imaging region is generated. The movement detection technology and the composite imaging technology may be combined to improve the resolution of the ultrasound image using the composite imaging technology, while ensuring the resolution of imaging of moving tissue regions such as the heart. Second, the variance of the Gaussian distribution may be roughly determined based on the speed level. The higher the speed level, the worse the correlation between two adjacent pieces of emission data, and the larger the variance of the Gaussian distribution. The lower the speed level, the better the correlation between two adjacent pieces of emission data, and the smaller the variance of the Gaussian distribution, which helps to determine a more reasonable composite weight sequence and reduce the impact of emission data with poor correlation on the ultrasonic imaging. Third, the movement detection is performed on the imaging region by emitting the plane wave to obtain the tissue movement speed of at least one pixel point in the imaging region, and then the sub-region is divided and the speed level of the sub-region is determined. The speed level of the tissue movement speed of the pixel point may be determined quickly and effectively, which improves the composition efficiency. Fourth, the imaging region is divided into at least one sub-region based on the frequency shift threshold and the relationship between the frequency shift and the tissue movement speed. When the tissue movement speed is uncertain, the sub-region may be divided and the corresponding speed level may be determined. Fifth, the plurality of initial frequency shifts are calculated based on the Doppler effect generated by the movement of the imaging region, and the average value of the plurality of initial frequency shifts is calculated, which may improve the accuracy of calculating the tissue movement speed and help to determine regions with faster tissue movement speed such as the myocardium. Sixth, when the target weight is assigned to the target beamforming result and weights on two sides of the target weight are assigned to beamforming results on two sides of the target beamforming result, the proportion of beamforming results of emissions close to the target emission in the composition may be increased, and the proportion of beamforming results of emissions far from the target emission in the composition may be reduced, thereby avoiding interference caused by excessive movement of tissues such as the heart and ensuring the resolution of the ultrasound image. Seventh, by selecting some elements in the beamforming result sequence and the composite weight sequence, determining the composite result and then determining the ultrasound image of the imaging region, better results may be obtained while reducing the amount of calculation.

Having thus described the basic concepts, it may be rather apparent to those skilled in the art after reading this detailed disclosure that the foregoing detailed disclosure is intended to be presented by way of example only and is not limiting. Although not explicitly stated here, those skilled in the art may make various modifications, improvements, and amendments to the present disclosure. These alterations, improvements, and amendments are intended to be suggested by this disclosure and are within the spirit and scope of the exemplary embodiments of the present disclosure.

Moreover, certain terminology has been used to describe embodiments of the present disclosure. For example, the terms "one embodiment," "an embodiment," and/or "some embodiments" mean that a particular feature, structure, or feature described in connection with the embodiment is included in at least one embodiment of the present disclosure. Therefore, it is emphasized and should be appreciated that two or more references to "an embodiment", "one embodiment", or "an alternative embodiment" in various portions of the present disclosure are not necessarily all referring to the same embodiment. In addition, some features, structures, or characteristics of one or more embodiments in the present disclosure may be properly combined.

Furthermore, the recited order of processing elements or sequences, or the use of numbers, letters, or other designations, therefore, is not intended to limit the claimed processes and methods to any order except as may be specified in the claims. Although the above disclosure discusses some embodiments of the invention currently considered useful by various examples, it should be understood that such details are for illustrative purposes only, and the additional claims are not limited to the disclosed embodiments. Instead, the claims are intended to cover all combinations of corrections and equivalents consistent with the substance and scope of the embodiments of the present disclosure. For example, although the implementation of various components described above may be embodied in a hardware device, it may also be implemented as a software only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. However, this disclosure does not mean that object of the present disclosure requires more features than the features mentioned in the claims. Rather, claimed subject matter may lie in less than all features of a single foregoing disclosed embodiment.

In some embodiments, the numbers expressing quantities or properties used to describe and claim certain embodiments of the present disclosure are to be understood as being modified in some instances by the term "about," "approximate," or "substantially." For example, "about," "approximate," or "substantially" may indicate $\pm 20\%$ variation of the value it describes, unless otherwise stated. Accordingly, in some embodiments, the numerical parameters set forth in the written description and attached claims are approximations that may vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the present disclosure are approximations, the numerical values set forth in the specific examples are reported as precisely as practicable.

Each of the patents, patent applications, publications of patent applications, and other material, such as articles, books, specifications, publications, documents, things, and/or the like, referenced herein is hereby incorporated herein by this reference in its entirety for all purposes. History application documents that are inconsistent or conflictive with the contents of the present disclosure are excluded, as well as documents (currently or subsequently appended to the present specification) limiting the broadest scope of the claims of the present disclosure. By way of example, should there be any inconsistency or conflict between the description, definition, and/or the use of a term associated with any of the incorporated material and that associated with the present document, the description, definition, and/or the use of the term in the present document shall prevail.

In closing, it is to be understood that the embodiments of the present disclosure disclosed herein are illustrative of the principles of the embodiments of the present disclosure. Other modifications that may be employed may be within the scope of the present disclosure. Thus, by way of example, but not of limitation, alternative configurations of the embodiments of the present disclosure may be utilized in accordance with the teachings herein. Accordingly, embodiments of the present disclosure are not limited to that precisely as shown and described.

**Claims**

1. A method for ultrasonic composite imaging, comprising:

 obtaining a beamforming result sequence of at least one pixel point in an imaging region, wherein the beamforming result sequence includes beamforming results that are arranged according to their respective emission orders;
 determining a composite weight sequence based on a tissue movement speed of the at least one pixel point; and
 determining, based on the beamforming result sequence and the composite weight sequence, a composite result to generate an ultrasound image of the imaging region.

2. The method of claim 1, wherein the determining a composite weight sequence based on a tissue movement speed of the at least one pixel point includes:

 determining a speed level of the tissue movement speed of the at least one pixel point; and
 determining, based on the speed level, the composite weight sequence.

3. The method of claim 2, wherein the determining a speed level of the tissue movement speed of the at least one pixel point includes:

 dividing, based on plane wave data in the imaging region, the imaging region into at least one sub-region and determining a speed level of the at least one sub-region; and
 determining the speed level of the sub-region where the at least one pixel point is located as the speed level of the tissue movement speed of the at least one pixel point.

4. The method of claim 3, wherein the dividing, based on plane wave data in the imaging region, the imaging region into at least one sub-region:

 determining a frequency shift of each of the at least one pixel point in the imaging region based on the plane wave data in the imaging region;
 presetting at least one frequency shift threshold; and
 dividing, based on the at least one frequency shift threshold and a proportional relationship between the frequency shift and the tissue movement speed, the imaging region into the at least one sub-region.

5. The method of claim 4, wherein the determining a frequency shift of each of the at least one pixel point in the imaging region based on the plane wave data in the imaging region includes:
for each pixel point,

 obtaining a plurality of pieces of plane wave data via a plurality of emissions and determining a plurality of initial frequency shifts based on the Doppler theorem; and
 determining an average value of the plurality of initial frequency shifts as the frequency shift of the pixel point.

6. The method of any one of claims 3 to 5, wherein the composite weight sequence is determined based on Gaussian distributions; and the determining, based on the speed level, the composite weight sequence includes:

 determining a variance of at least one of the Gaussian distributions based on the speed level of the at least one sub-region;
 generating the composite weight sequence of the at least one sub-region based on the variance of the at least one Gaussian distribution; and
 determining the composite weight sequence of the at least one pixel point according to the sub-region where the at least one pixel point is located.

7. The method of claim 6, wherein the variance of the at least one Gaussian distribution is positively correlated with the speed level.

8. The method of any one of claims 2 to 7, wherein the determining a speed level of the tissue movement speed of the at least one pixel point further includes:

obtaining a plurality of reference ultrasound images by emitting a focused wave targeted at the imaging region;

determining tissue movement information between adjacent reference ultrasound images based on a correspondence between the adjacent reference ultrasound images;

dividing the imaging region into at least one sub-region based on the tissue movement information and determining a speed level of the at least one sub-region; and

determining the speed level of the tissue movement speed of the at least one pixel point based on a sub-region, among the at least one sub-region, where the at least one pixel point is located.

9. The method of any one of claims 1 to 8, wherein the determining, based on the beamforming result sequence and the composite weight sequence, a composite result to generate an ultrasound image of the imaging region includes:

determining a target beamforming result based on the beamforming result sequence, wherein the target beamforming result is a beamforming result in the beamforming result sequence that satisfies a first preset condition;

determining a target weight in the composite weight sequence based on a Gaussian distribution, wherein the target weight is a weight that satisfies a second preset condition;

determining a weight allocation result by allocating the target weight and weights on two sides of the target weight to the target beamforming result and beamforming results on two sides of the target beamforming result, respectively; and

determining the composite result by compositing the beamforming result sequence based on the weight allocation result, and generating the ultrasound image based on the composite result.

10. The method of claim 9, wherein the beamforming result in the beamforming result sequence that satisfies a first preset condition includes a beamforming result in the beamforming result sequence that corresponds to an emission perpendicular to the at least one pixel point.

11. The method of any one of claims 1 to 10, wherein the obtaining a beamforming result sequence of at least one pixel point in an imaging region includes:

emitting a focused wave targeted at the imaging region for multiple times; and

for each pixel point in the at least one pixel point,

determining at least one beamforming result based on at least one emission of the focused wave, and

generating the beamforming result sequence of the pixel point based on the at least one beamforming result.

12. A system for ultrasonic composite imaging, comprising:

an acquisition module, configured to obtain a beamforming result sequence of at least one pixel point in an imaging region, wherein the beamforming result sequence includes beamforming results that are arranged according to their respective emission orders;

a determination module, configured to determine a composite weight sequence based on a tissue movement speed of the at least one pixel point; and

a generation module, configured to determine, based on the beamforming result sequence and the composite weight sequence, a composite result to generate an ultrasound image of the imaging region.

13. The system of claim 12, wherein the determination module is further configured to:

determine a speed level of the tissue movement speed of the at least one pixel point; and

determine, based on the speed level, the composite weight sequence.

14. The system of claim 13, wherein the determination module is further configured to:

divide, based on plane wave data in the imaging region, the imaging region into at least one sub-region and determine a speed level of the at least one sub-region; and

determine the speed level of the sub-region where the at least one pixel point is located as the speed level of the tissue movement speed of the at least one pixel point.

15. The system of claim 14, wherein the determination module is further configured to:

determine a frequency shift of each of the at least one pixel point in the imaging region based on the plane wave data in the imaging region;

preset at least one frequency shift threshold; and

divide, based on the at least one frequency shift threshold and a proportional relationship between the frequency shift and the tissue movement speed, the imaging region into the at least one sub-region.

16. The system of claim 15, wherein the determination module is further configured to:

for each pixel point,

obtain a plurality of pieces of plane wave data via a plurality of emissions and determine a plurality of initial frequency shifts based on the Doppler theorem; and

determine an average value of the plurality of initial frequency shifts as the frequency shift of the pixel point.

17. The system of any one of claims 14 to 16, wherein the composite weight sequence is determined based on Gaussian distributions; and the determination module is further configured to:

determine a variance of at least one of the Gaussian distributions based on the speed level of the at least one sub-region;

generate the composite weight sequence of the at least one sub-region based on the variance of the at least one Gaussian distribution; and

determine the composite weight sequence of the at least one pixel point according to the sub-region where the at least one pixel point is located.

18. The system of claim 17, wherein the variance of the at least one Gaussian distribution is positively correlated with the speed level.

19. The system of any one of claims 13 to 18, wherein the determination module is further configured to:

obtain a plurality of reference ultrasound images by emitting a focused wave targeted at the imaging region;

determine tissue movement information between adjacent reference ultrasound images based on a correspondence between the adjacent reference ultrasound images;

divide the imaging region into at least one sub-region based on the tissue movement information and determine a speed level of the at least one sub-region; and

determine the speed level of the tissue movement speed of the at least one pixel point based on a sub-region, among the at least one sub-region, where the at least one pixel point is located.

20. The system of any one of claims 12 to 19, wherein the generation module is further configured to:

determine a target beamforming result based on the beamforming result sequence, wherein the target beamforming result is a beamforming result in the beamforming result sequence that satisfies a first preset condition;

determine a target weight in the composite weight sequence based on a Gaussian distribution, wherein the target weight is a weight that satisfies a second preset condition;

determine a weight allocation result by allocating the target weight and weights on two sides of the target weight to the target beamforming result and beamforming results on two sides of the target beamforming result, respectively; and

determining the composite result by compositing the beamforming result sequence based on the weight allocation result, and generating the ultrasound image based on the composite result.

21. The system of claim 20, wherein the beamforming result in the beamforming result sequence that satisfies a first preset condition includes a beamforming result in the beamforming result sequence that corresponds to an emission perpendicular to the at least one pixel point.

22. The system of any one of claims 12 to 21, wherein the acquisition module is further configured to:

emit a focused wave targeted at the imaging region for multiple times; and

for each pixel point in the at least one pixel point,

determine at least one beamforming result based on at least one emission of the focused wave, and

generate the beamforming result sequence of the pixel point based on the at least one beamforming result.

23. A device for ultrasonic composite imaging, comprising at least one memory and at least one processor; the at least one memory storing computer instructions that, when executed or partially executed by the at least one processor, cause the at least one processor to perform the method of any one of claims 1 to 11.

24. A non-transitory computer-readable storage medium storing computer instructions that, when read by a computer, cause the computer to perform the method of any one of claim 1-11.

**100**

**FIG. 1**

**200**

Acquisition module
210

Determination
module
220

Generation module
230

**FIG. 2**

**300**

Obtaining a beamforming result sequence of at least one pixel point in an imaging region ⟋ 310

Determining a composite weight sequence based on a tissue movement speed of the at least one pixel point ⟋ 320

Determining, based on the beamforming result sequence and the composite weight sequence, a composite result to generate an ultrasound image of the imaging region ⟋ 330

**FIG. 3**

**FIG. 4**

**FIG. 5**

(A single focused wave emission)

Pixel point a

Angle $\gamma^\circ$

Effective imaging region

Focal position

Synthesize echo signal

A beamforming result sum corresponding to a focused wave emission of pixel point a

**FIG. 6**

**FIG. 7**

**800**

Determining a speed level of a tissue
movement speed of at least one pixel point    810

Determining a composite weight sequence
based on the speed level    820

**FIG. 8**

FIG. 9

<u>**1000**</u>

```
┌─────────────────────────────────────┐
│ Determining a target beamforming     │    ⌐1010
│ result based on a beamforming        │
│ result sequence                      │
└─────────────────────────────────────┘
                  │
                  ▼
┌─────────────────────────────────────┐
│ Determining a target weight in a     │    ⌐1020
│ composite weight sequence based on   │
│ a Gaussian distribution              │
└─────────────────────────────────────┘
                  │
                  ▼
┌─────────────────────────────────────┐
│ Determining a weight allocation      │    ⌐1030
│ result by allocating the target      │
│ weight and weights on two sides of   │
│ the target weight to the target      │
│ beamforming result and beamforming   │
│ results on two sides of the target   │
│ beamforming result, respectively     │
└─────────────────────────────────────┘
                  │
                  ▼
┌─────────────────────────────────────┐
│ Determining a composite result by    │    ⌐1040
│ compositing the beamforming result   │
│ sequence based on the weight         │
│ allocation result, and generating    │
│ an ultrasound image based on the     │
│ composite result                     │
└─────────────────────────────────────┘
```

**FIG. 10**

Movement detection sequence

Imaging sequence

1110

Obtaining a frequency shift of at least one pixel point in an imaging region

1130

1140

Obtaining a beamforming result sequence of the at least one pixel point

1120

Dividing the imaging region based on the frequency shift of the at least one pixel point and a proportional relationship between the frequency shift and a tissue movement speed

Determining a speed level of the tissue movement speed of the at least one pixel point based on a sub-region where the pixel point is located, and determining a composite weight sequence corresponding to the sub-region

1150

Composition

1160

Generating an ultrasound image of the imaging region

**FIG. 11**

| Operating system |
| Computer program |
| Database |
| Non-volatile storage medium |

Processor

Internal memory

System bus

Input and output interface

Network interface

**Computer device**

**FIG. 12**

## INTERNATIONAL SEARCH REPORT

| International application No. |
| --- |
| **PCT/CN2023/131868** |

### A. CLASSIFICATION OF SUBJECT MATTER

A61B8/00(2006.01)i

According to International Patent Classification (IPC) or to both national classification and IPC

### B. FIELDS SEARCHED

Minimum documentation searched (classification system followed by classification symbols)

IPC: A61B8/-

Documentation searched other than minimum documentation to the extent that such documents are included in the fields searched

Electronic data base consulted during the international search (name of data base and, where practicable, search terms used)

CNTXT, ENTXT, ENTXTC, CNKI: 超声, 图像, 复合, 运动, 权重, 运动, 多普勒, 像素, 频率, 频移, ultrasound, image, composite, motion, weights, motion, doppler, pixel, frequency, frequency shift

### C. DOCUMENTS CONSIDERED TO BE RELEVANT

| Category* | Citation of document, with indication, where appropriate, of the relevant passages | Relevant to claim No. |
| --- | --- | --- |
| PX | CN 115721337 A (WUHAN UNITED IMAGING HEALTHCARE CO., LTD.) 03 March 2023 (2023-03-03) <br> claims 1-13 | 1-24 |
| X | CN 110536647 A (COVIDIEN LP) 03 December 2019 (2019-12-03) <br> description, paragraphs [0044]-[0084], and figures 1-5 | 1-3, 6-14, 17-24 |
| Y | CN 110536647 A (COVIDIEN LP) 03 December 2019 (2019-12-03) <br> description, paragraphs [0044]-[0084], and figures 1-5 | 4, 5, 15, 16 |
| Y | CN 104105449 A (MAUI IMAGING, INC.) 15 October 2014 (2014-10-15) <br> description, paragraphs [0053]-[0076], and figures 1-21 | 4, 5, 15, 16 |
| A | CN 102727255 A (EDAN INSTRUMENTS INC.) 17 October 2012 (2012-10-17) <br> entire document | 1-24 |
| A | CN 112826533 A (SHENZHEN WISONIC MEDICAL TECHNOLOGY CO., LTD.) 25 May 2021 (2021-05-25) <br> entire document | 1-24 |

☑ Further documents are listed in the continuation of Box C.    ☑ See patent family annex.

| | |
| --- | --- |
| * Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
| --- | --- |
| **17 December 2023** | **21 December 2023** |

| Name and mailing address of the ISA/CN | Authorized officer |
| --- | --- |
| **China National Intellectual Property Administration (ISA/ CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2023/131868** |

**C. DOCUMENTS CONSIDERED TO BE RELEVANT**

| Category* | Citation of document, with indication, where appropriate, of the relevant passages | Relevant to claim No. |
| --- | --- | --- |
| A | US 5655535 A (SIEMENS MEDICAL SYSTEMS, INC.) 12 August 1997 (1997-08-12)<br>entire document | 1-24 |
| A | US 6759954 B1 (HUBBELL INCORPORATED) 06 July 2004 (2004-07-06)<br>entire document | 1-24 |

Form PCT/ISA/210 (second sheet) (July 2022)

**INTERNATIONAL SEARCH REPORT**
Information on patent family members

International application No.

**PCT/CN2023/131868**

| Patent document cited in search report | | | Publication date (day/month/year) | Patent family member(s) | | | Publication date (day/month/year) |
|---|---|---|---|---|---|---|---|
| CN | 115721337 | A | 03 March 2023 | None | | | |
| CN | 110536647 | A | 03 December 2019 | US | 2019369240 | A1 | 05 December 2019 |
| | | | | WO | 2018145293 | A1 | 16 August 2018 |
| CN | 104105449 | A | 15 October 2014 | EP | 2785253 | A1 | 08 October 2014 |
| | | | | EP | 2785253 | B1 | 15 November 2023 |
| | | | | JP | 2015500062 | A | 05 January 2015 |
| | | | | JP | 6407719 | B2 | 17 October 2018 |
| | | | | HK | 1202401 | A1 | 02 October 2015 |
| | | | | KR | 20140098843 | A | 08 August 2014 |
| | | | | WO | 2013082455 | A1 | 06 June 2013 |
| | | | | US | 2013144166 | A1 | 06 June 2013 |
| | | | | US | 10226234 | B2 | 12 March 2019 |
| | | | | US | 2019200961 | A1 | 04 July 2019 |
| | | | | US | 11826204 | B2 | 28 November 2023 |
| | | | | TW | 201336478 | A | 16 September 2013 |
| CN | 102727255 | A | 17 October 2012 | None | | | |
| CN | 112826533 | A | 25 May 2021 | None | | | |
| US | 5655535 | A | 12 August 1997 | None | | | |
| US | 6759954 | B1 | 06 July 2004 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202211487363